# EUROPÄISCHE PATENTSCHRIFT

(11) **EP 0 843 591 B1**
(45) Veröffentlichungstag und Bekanntmachung des Hinweises auf die Patenterteilung: **07.03.2001**
(21) Anmeldenummer: 96918662.6
(22) Anmeldetag: 03.06.1996
(51) Int. Cl.: B01J 13/00, C08F 16/06, G01N 33/546, B01D 15/08, C07H 1/06, C12Q 1/68

(54) **MAGNETISCHE POLYMERPARTIKEL AUF DER BASIS VON POLYVINYLALKOHOL, VERFAHREN FÜR IHRE HERSTELLUNG UND VERWENDUNG**
POLYVINYL ALCOHOL-BASED MAGNETIC POLYMER PARTICLES, METHODS FOR THEIR PREPARATION AND THEIR USE
PARTICULES POLYMERES MAGNETIQUES A BASE D'ALCOOL DE POLYVINYLE, PROCEDES PERMETTANT DE LES PRODUIRE ET UTILISATION

(30) Priorität: 31.07.1995 DE 19528029
(43) Veröffentlichungstag der Anmeldung: 27.05.1998
(73) Patentinhaber: Müller-Schulte, Detlef, 52074 Aachen (DE)
(72) Erfinder: Müller-Schulte, Detlef, 52074 Aachen (DE)
(74) Vertreter: Flaccus, Rolf-Dieter, Dr.
(86) Internationale Anmeldenummer: EP9602398
(87) Internationale Veröffentlichungsnummer: WO9704862

(56) Entgegenhaltungen:
- DE-A- 4 127 657
- CHEMICAL ABSTRACTS, Band 115, Nr. 26, ver!ffentlicht 1991, 30 Dezember, (Columbus, Ohio, USA), H. YOKOI "Synthesis of poly- mer membranes homogeneously dispersed with ultra-fine magnetic particles as assisted by techniques of plasma chemistry", Seiten 34-35, Nr. 281 068h; & Kenkyu Hokoku - Asahi Garasu Zaidan 1990, 57, 143-51.

## Beschreibung

Gegenstand der vorliegenden Erfindung sind Verfahren zur Herstellung perl- bzw. kugelförmiger Polymerpartikel (Beads) auf der Basis von Polyvinylalkohol (PVAL), in die ein magnetisches Kolloid eingekapselt ist, das den Polymerpartikeln magnetische Eigenschaften verleiht und dazu befähigt, Biomoleküle oder Zellen zu binden.

Magnetische Polymerpartikel wurden in den letzten Jahren vor allem in der Biochemie und Medizin vornehmlich zur Abtrennung von Zellen, Proteinen und Nukleinsäuren verwendet. Aufgrund der magnetischen Eigenschaften lassen sie sich auch als Transportsysteme für bestimmte Pharmaka in bestimmte Körperareale nutzen. Der Einsatz von Magnetpartikeln bietet von der praktischen Handhabung her große Vorteile gegenüber herkömmlichen Separationsystemen, da die meist als feine Suspensionen oder Emulsionen vorliegenden Magnetpartikel mittels magnetischer Kräfte aus der Mischung abgetrennt werden können. Diese Abtrenntechnik macht die übliche Zentrifugation überflüssig. Die magnetische Fraktion kann ferner innerhalb einer Minute abgetrennt werden, was im Vergleich zu den herkömmlichen chromatographischen Säulentrenntechniken eine erhebliche Zeitersparnis bedeutet. Bei letzterer Technik fallen vor allem das zeitaufwendige Equilibrieren und Eluieren ins Gewicht, Prozesse also, die bei der Magnet-Bead-Technik praktisch entfallen. Ein weiterer wesentlicher Vorteil, die die Magnet-Bead-Technologie auszeichnet, besteht in der Art der Reaktionskinetik. Bei den Füllmedien für die Säulenchromatographie greift man in der Regel auf Korngrößen von 50 - 100 µm zurück. Da bei solchen Korngrößen die Trennkapazitäten jedoch vielfach nicht ausreichen, geht der Trend zunehmend dahin, Korngrößen von < 50 µm und sogar < 10 µm anzuwenden. Damit die beim Säulendurchlauf erzeugten hohen Drucke widerstanden werden können, weisen solche Medien praktisch keine Porositäten mehr auf, weshalb man in praxi von den durchsichtigen Kunststoff- bzw. Glassäulen zu druckstabilen Stahlsäulen übergehen mußte. Die dafür erforderlichen leistungsfähigen Pumpsysteme sind ein weiterer Nachteil der heutigen Säulenchromatographie-Technik. Diese Nachteile, die letztlich in der ungenügenden Umsatzkinetik begründet sind, können durch die Anwendung der Magnet-Bead-Technologie völlig umgangen werden.

Durch Verwendung feindisperser PVAL Partikel, die eine Teilchengröße von 1-10 µm, vorzugsweise eine solche von 1-4 µm, aufweisen, verbleiben die Teilchen über mehrere Stunden hinweg im suspendierten Zustand, so dass die Umsatzkinetik der einer quasi-homogenen Lösung entspricht. Infolge der stabilen Suspension kann auch in den meisten Fällen auf Rühren oder Schütteln verzichtet werden.

Verfahren zur Herstellung von magnetischen Eisen-Dextran Mikropartikeln sind in der U.S. Patentschrift 4,452,773 beschrieben. Durch Mischen einer Fe(II)- und Fe(III)-Salzlösung in Gegenwart einer definierten Menge Dextran und anschließender Zugabe von Alkali werden 30-40 nm große kolloidale Fe-Oxid Teilchen gewonnen, an die Dextran adsorbiert ist. Ein ähnliches Verfahren liegt der PCT Anmeldung WO 90/07380 zugrunde. Es werden Fe(II)- und Fe(III)-Salzlösungen unter Zugabe von Dextran bei 40°C behandelt und anschließend mit NaOH titriert, aufgrund dessen superparamagnetische Partikel mit einer Größe von 40-100 nm erhalten werden. Der Nachteil beider Verfahren im Hinblick auf rasche und einfache Abtrennung, besteht darin, daß aufgrund der Feinheit der Partikel eine Separation nur mittels eines hochgradienten Magnetfeldes (High Gradient Magnetic Field) möglich ist. Dieses hochgradiente Magnetfeld wird durch eine Trennsäule, die mit Stahlwolle oder ähnlichen mikropartikulären Substanzen dicht gefüllt ist, und sich zwischen den Polschuhen zweier starker Elektro- bzw. Handmagnete befindet, erzeugt. Die Trennung der Teilchen erfolgt durch Hindurchleiten der Suspension durch die gefüllte Trennsäule. Eine Abtrennung solcher Kolloide ist mittels herkömmlicher Handmagnete nicht möglich. Grundsätzliche experimentelle Unterschiede zu der herkömmlichen Chromatographie-Technik bestehen somit kaum. Ein weiterer Nachteil der zitierten Herstellungsverfahren ist, daß durch den eigentlichen Herstellungsprozess keine einheitlichen Teilchengrößen gewonnen werden, vielmehr werden diese erst durch eine fraktionierte Magnetseparation erhalten. Erschwerend für den Nachweis dieser Magnetpartikel ist ferner der Umstand, daß die Teilchen unter dem Lichtmikroskop nicht mehr sichtbar sind. In einem weiteren Verfahren, das dem U.S. Patent 4,070,246 zugrunde liegt, werden Magnetpartikel durch Umsetzen von p-Aminobenzoesäure und einem Aldehyd unter Zugabe eines ferromagnetischen Pulvers gewonnen. Die Herstellung definierter perlförmiger Partikel, wie sie für diagnostische Tests erforderlich sind, ist mit diesem Verfahren nicht möglich. Chemische Kopplungen von Biomolekülen an den Träger sind ebenfalls nicht möglich. Gleiches gilt auch für die in den U.S. Patentschriften 4,106,448, 4,136,683 und 4,735,796 beschriebenen Verfahren zur Herstellung von Dextran eingekapselten magnetischen Partikeln für die Diagnostik und für die Tumortherapie. Auch bei den vorgenannten Verfahren sind kovalente Kopplungen von Biomolekülen nicht beschrieben. In der U.S. Patentschrift 4,647,447 wird die Herstellung ferromagnetischer Partikel für die NMR-Diagnostik beschrieben. Hierbei wird entweder von Fe(II)/Fe(III)-Salzlösungen oder direkt von mikropartikulären Ferriten ausgegangen, die in Gegenwart eines Komplexbildners in Form von Serum Albumin, Polysacchariden, Dextran oder Dextrin zu Magnetsuspensionen umgesetzt werden. Weitere ferromagnetische Partikel, die in einer Silan-Matrix eingekapselt sind, werden in der U.S. Patentschrift 4,628,037 behandelt. Ebenfalls als Kontrastmittel für die NMR-Diagnostik dienen superparamagnetische Eisenoxide, die in dem U.S. Patent 4,827,945 behandelt werden. Durch Ausfällen von Fe(II)/Fe(III)-Salzmischungen mittels Basen in Gegenwart von Serum Albumin, Polypeptiden oder Polysacchariden lassen sich mit diesen Substanzen beschichtete Magnetpartikel herstellen. Durch Ankopplung bestimmter Antikörper an die Matrix können die Magnetteilchen in bestimmte Körperareale dirigiert werden (Targeting). Die Herstellung von Eisenoxiden durch Ausfällen von Eisensalzen in Gegenwart von z.B. Dextranen oder Polyglutaraldehyden liegt den U.S. Patenten 2,870,740 und 4,267,234 zugrunde. Allen vorgenannten Verfahren und Produkten ist gemeinsam, daß die ferromagnetischen oder superparamagnetischen Partikel erst durch Ausfällen einer Fe-Salzlösung, die ein bestimmtes molares Verhältnis von Fe(II) und Fe(III)-Salzen voraussetzt, in Gegenwart eines Komplexbildners bzw. Beschichtungsagens hergestellt werden. Die beschriebenen Teilchen weisen eine mehr oder weniger breite Korngrößenverteilung auf. Definierte perl- bzw. kugelförmige Partikel, lassen sich mit den vorgenannten Verfahren nicht herstellen. Die beschriebenen Mittel weisen eine mehr oder weniger amorphe geometrische Struktur auf. Aufgrund ihrer Feinheit, die durchweg im nm-Bereich liegt, eignen sie sich daher vorwiegend als Kontrastmittel für die NMR-Diagnostik oder als Zell-Markierungsmittel (Zellmarker). Die Abtrennung der magnetischen Fraktionen sind darüberhinaus in den meisten Fällen mittels einfacher Handmagnete, wie sie für schnelle diagnostische Tests oder affinitätschromatographische Abtrennungen vorteilhaft sind, nicht möglich.

Die Präparation magnetischer Albumin- bzw. Protein-Mikropartikel, die mit bestimmten Bindungsagenzien beschichtet sind und für die Virus- und Zell-Separationen sowie diagnostische Tests eingesetzt werden können, sind in den U.S. Patenten 4,345,588; 4,169,804; 4,115,534 ; 4,230,685; 4,247,406 und 4,357,259 beschrieben.

Magnetpartikel mit einer definierten perlförmigen Struktur sind aus den U.S. Patenten 4,861,705 bekannt. Gegenstand des vorgenannten Patentes sind Agarose-Polyaldehyd-Komposit-Partikel, die durch Suspension der Polymerphase in einer ÖlPhase gewonnen werden. Durch Zumischen eines Ferrofluides, dies sind definitionsgemäß sehr feine superparamagnetische, wäßrige Eisenoxide-Kolloide, zu der Polymerphase werden magnetische Polymerpartikel mit einer Teilchengröße von 40-1000 µm erhalten.

Ideal-kugelförmig Partikel werden in dem U.S. Patent 4,654,267 beschrieben. Das Verfahren unterscheidet sich grundsätzlich von den vorgenannten dadurch, daß Polyacrylate oder Polysstyrol als Matrix verwendet werden, die zunächst mittels Suspensionspolymerisation zu perlförmigen Partikeln radikalisch polymerisiert werden. Anschließend werden die Teilchen in einer organischen Phase unter definierten Bedingungen gequollen. Es folgt eine Inkubation der Polymerpartiklel in einer Fe(II)/Fe(III)-Salzlösung, die, nachdem sie in die Teilchen diffundiert ist, mittels Ammoniak zu superparamagnetischen Eisenoxiden oxidiert werden. Das Verfahren liefert perlförmige Partikel mit Korngrößen zwischen 0.5 und 20 µm. Das Verfahren selber ist technisch sehr aufwendig. Neben der Verwendung hochtoxischer Substanzen beträgt der Zeitaufwand für die Präparation der Grundmatrix 10-30 Stunden. Ferner bedarf es zusätzlicher Nitro-, Nitroso- oder Amino-Gruppen, die durch einen zusätzlichen Präparationsschritt in die Polymermatrix eingeführt wird, um eine ausreichende Absorption der Fe-Salze zu gewährleisten. Der entscheidende Nachteil der dort beschriebenen Partikel liegt in dem Grundpolymer Polystyrol begründet. Polystyrol stellt ein äußerst hydrophobes Material dar, das im Kontakt mit Proteinlösungen oder anderen Biomolekülen stark zur unspezifischen Adsorption neigt, ein Phänomen, das besonders bei Immunoassays und affinitätschromatographischen Auftrennungen nachteilig ist. Die Nachteile der vorgenannten Verfahren in Bezug auf Herstellungsaufwand, Partikelgeometrie, magnetisches Separationsverhalten, Eigenschaften der Polymermatrix oder Art der Kopplungsweise können durch ein neuartiges Wasserin-Öl-Suspensionsverfahren umgangen werden.

Als Polymermatrix wird Polyvinylalkohol (PVAL) verwendet, der als wäßrige Lösung in einer organischen, nicht mit Wasser mischbaren Phase unter Rühren suspendiert und vernetzt wird. Beispiele für solche organischen Phasen sind allgemein aus dem Stand der Technik der Suspensionspolymerisation bekannt. Vorzugsweise werden für das erfindungsgemäße Verfahren handelsübliche Pflanzenöle verwendet. Um zu den gewünschten magnetischen Eigenschaften der Polymerpartikel zu gelangen, wird die Polymerphase vor der Suspension mit einem magnetischen Kolloid z.B. in Form von Eisenoxid-Pulvern oder Ferrofluiden vermischt und anschließend in der Ölphase suspendiert. Die Herstellung perlförmiger PVAL Partikel durch Suspension einer wäßrigen Polymerlösung ist in der DE-OS 4127657 beschrieben. Durch Zugabe von Magnetit-Pulver zur Polymerlösung können magnetische Partikel hergestellt werden. Das dort beschriebene Verfahren verwendet Polymerlösungen und Ölphasen, die keine Zusätze in Form von Emulgatoren oder sonstigen oberflächenaktiven Substanzen enthält. Dadurch bedingt, liegen die Partikelgrößen durchweg zwischen 50 und 500 µm. Die Korngrößen werden bei dem vorgenannten Verfahren in erster Linie durch die Viskosität der organischen- und/oder der Polymer-Phase bestimmt.

Das Ziel der vorliegenden Erfindung sind demgegenüber Magnetpartikel, die eine Partikelgröße im Bereich von 1-10 µm, vorzugsweise zwischen 1-4 µm aufweisen und darüberhinaus eine sehr enge Korngrößenverteilung aufweisen. Nur solche Teilchen lassen sich für die Zellseparation/-markierung, die Aufreinigung von Biosubstanzen in Suspension sowie für diagnostische Assays einsetzen.

Es wurde überraschenderweise gezeigt, daß solche Polymer Partikel durch Zugabe von bestimmten Emulgator-Mischungen zu der Ölphase realisiert werden können. Der Begriff Emulgator wird im folgenden definitionsgemäß als Oberbegriff für alle oberflächenaktiven Substanzen wie Tenside, Detergenzien oder Suspensionsstabilisatoren verwendet. Emulgatoren, die sich als Zusätze für die Ölphase eignen sind z.B.: Propylenoxid-Äthylenoxid-Blockcopolymere, Sorbitan-Fettsäureester, Komplexmischester aus Pentaerythrit-Fettsäureester mit Citronensäure, Polyäthylenglykol-Castoröl-Derivate, Blockcopolymere aus Rizinusöl-Derivaten, Polyäthylenglykole, modifizierte Polyester, Polyoxyäthylen-Sorbitan-Fettsäureester, Polyoxyäthylen-Polyoxypropylen-Äthylendiamin-Blockcopolymere, Polyglyceryl-Derivate, Polyoxyäthylen-Alkohol-Derivate, Alkylphenylpolyäthylenglykol-Derivate, Polyhydroxyfettsäure-Polyäthylenglykol-Blockcopolymere, Polyäthylenglykol-Ätherderivate. Substanzen dieser Art sind im Handel u.a. unter der Handelsbezeichnung: Pluronic®, Synperonic®, Tetronic®, Triton®, Arlacel®, Span®, Tween®, Brij®, Renex®, Hypermer®, Lameform®, Dehymuls® oder Eumulgin® bekannt.

Im Hinblick auf einheitliche, perlförmige Polymer Partikel mit den geforderten Partikelgrößen von 1-10 µm hat sich überraschenderweise gezeigt, daß für die Ölphase nur eine Mischung aus mindestens zwei, vorzugsweise drei bis vier oberflächenaktiven Substanzen, zu der geforderten Partikelspezifikation führt. Voraussetzung für die Realisierung der geforderten Partikelgrößen ist eine entsprechende Verringerung der Grenzflächenspannung der Phasen. Dies wird überraschenderweise durch Mischen von einer lipohilen Emulgatorkomponente mit mindestens einem Emulgator ermöglicht, der semi-hydrophile Eigenschaften aufweist, d.h. der sowohl wasser- als auch öllöslich ist. Emulgatoren, die die letzteren Eigenschaften erfüllen sind z.B.: Äthylenoxid-Propylenoxid-Blockcopolymer-Derivate mit überwiegendem Äthylenoxid-Anteil, Polyäthylenglykolhexadecyläther, kürzerkettige Polyoxyäthylen-Sorbitan-Fettsäureester, Polyäthylenglykole oder kürzerkettige Sorbitan-Fettsäureester.

Die Konzentration der Emulgatoren in der Ölphase beträgt in der Regel 2-6 Vol.%, vorzugsweise 3.5-5.0 Vol%. In Bezug auf Feinheit und enge Korngrößenverteilung der Polymertröpfchen sind solche Emulgatormischungen von Vorteil, die mindestens zwei lipophile Komponenten und einen semi-hydrophilen Emulgator enthalten. Die Konzentration des semi-hydrophilen Emulgators liegt in der in der Regel zwischen 15 und 30 Vol%, bezogen auf die Gesamt-Emulgatormenge. Die erfindungsgemäßen Verfahren ermöglichen neben der Feinheit der Teilchen auch die Herstellung perlförmiger Teilchen, die Voraussetzung für eine homogene Suspension sind. Dadurch wird exaktes Pipettieren, wie in der biochemischen-medizinischen Analytik/Diagnostik erforderlich, ermöglicht.

Neben den Emulgatoren für die Ölphase tragen auch spezielle oberflächenaktive Substanzen, die in der wäßrigen Polymerphase löslich sind, zur Verbesserung der Suspensionsqualität vor allem von Polymerlösungen mit niedrigen Molmassen (20.000-80.000) bei. Darüberhinaus hat sich überraschenderweise gezeigt, daß sich die in fester Form zugesetzten magnetischen Kolloide erst durch Zugabe ionischer Emulgatoren fein dispergieren lassen. Beispiele für solche Emulgatoren, die auch als binäre Mischungen eingesetzt werden können, sind: Serum Albumin, Gelatine, aliphatische und aromatische Sulfonsäure-Derivate, Polyäthylenglykole, Poly-N-Vinylpyrrolidon oder Cellulose-acetat-butyrat. Die Mengen der eingesetzten Emulgatoren betragen in der Regel 0.01 - 2 % Gew%, bezogen auf die Polymerphase, wobei die Konzentration der ionischen Emulgatoren durchweg zwischen 0.01 und 0.05 Gew% liegt. Die Einflüsse wie Rührgeschwindigkeit sowie Konzentration und Viskosität der beiden Phasen auf die Teilchengröße, wie in der DE-OS 4127657 gezeigt, spielen beim erfindungsgemäßen Verfahren aufgrund der Emulgatorzusätze nur eine untergeordnete Rolle. Zur Realisierung der erforderlichen Teilchengrößen von 1-10 µm reichen Rührgeschwindigkeiten von 1500-2000 U/Min. aus, wobei herkömmliche Zweiblatt-Propellerrührer zum Einsatz kommen. Der determinierende Einfluß der Emulgatoren des vorliegenden Verfahrens auf die Teilchengröße wird daraus ersichtlich, daß bei Erniedrigung der Rührgeschwindigkeit von 2000 auf 1300 U/Min. die Teilchengrößen von zuvor 1-5 µm auf 2-8 µm ansteigen. Bei Erhöhung der Rührgeschwindikeit von 2000 auf 5000 U/Min. ist praktisch keine Teilchengrößenveränderung festzustellen. Demgegenüber variieren die Teilchengrößen der nach der vorgenannten Methode hergestellten Partikel bei analoger Änderung der Rührgeschwindigkeiten zwischen 10 und 80 µm. Auch der in der vorgenannten Patentschrift beobachtete Einfluß der Viskositäten sowohl der Suspensionsphase als auch der Polymerphase auf die Teilchengrößen wirken sich bei dem erfindungsgemäßen Verfahren gegenüber dem Einfluß der Emulgatoren nur minimal aus. So schwanken die Teilchengrößen der PVAL-Partikel bei Veränderung der Viskosität der Polymerphase von 10 auf 1000 mPa s bei dem erfindungsgemäßen Verfahren nur zwischen 2 und 8 µm, beim vorgenannten Verfahren unter sonst gleichen Bedingungen dagegen zwischen 50 und 140 µm.

Als Magnetpartikel, die während des Suspensions-Vernetzungsprozesses in die Polymermatrix eingekapselt werden, können grundsätzlich solche ferro- oder superparamagnetischen Kolloide verwendet werden, die eine entsprechende Partikelgröße aufweisen und in der Regel über eine magnetische Sättigung von 50-400 Gauss verfügen.

Ein weitere Forderung, die die Magnetpartikel erfüllen müssen, ist die Dispergierbarkeit in der wäßrigen Polymerphase. Im Gegensatz zu dem im U.S. Patent 4,654,267 beschriebenen Verfahren zur Erzeugung magnetischer Partikel, dem ein aufwendiger Quellungsprozess mit Eisensalzen und anschließender Oxidation zu Magnetit-Kolloiden zugrunde liegt, können bei dem vorliegenden Verfahren die magnetischen Kolloide direkt in der Polymerphase dispergiert werden. Bei der anschließenden Suspension in der organischen Phase werden die Magnet-Kolloide dann simultan in den Polymertröpfchen eingeschlossen. Diese Verfahrensweise stellt eine deutliche verfahrenstechnische Vereinfachung gegenüber dem vorgenannten Verfahren dar, womit auch eine erhebliche Zeitersparnis für den Herstellungsprozess verbunden ist. Während zur Herstellung der vorgenannten Mittel auf Polystyrolbasis Präparationszeiten von 10 bis 30 Stunden erforderlich sind, benötigt man bei dem erfindungsgemäßen Verfahren lediglich 5- 30 Minuten zur Gewinnung der Basis-Magnetpartikel.

Als magnetische Kolloide kommen vorzugsweise Magnetite mit Partikelgrößen von 10-200 nm in Frage, wobei das erfindungsgemäße Verfahren nicht.auf diese Verbindungsklasse beschränkt ist. Solche Substanzen sind z.B. unter der Handelsbezeichnung Bayferrox oder Ferrofluidics im Handel erhältlich. Da die Herstellung solcher Kolloide allgemeiner Stand der Technik ist, können die Magnetteilchen auch nach den bekannten Verfahren, wie z.B. von Shinkai et al., Biocatalysis, Vol. 5, 1991, 61, Reimers und Khalafalla, Br. Patent 1,439,031 oder Kondo et al., Appl. Microbiol. Biotechnol., Vol 41, 1994, 99, beschrieben, hergestellt werden. Die Konzentrationen der Kolloide in der Polymerphase liegen, jeweils bezogen auf die Polymephase, in der Regel zwischen 4 und 14 Vol% bei den Kolloiden, die herstellungsbedingt bereits als wäßrige Kolloide vorliegen, und 0.3-2 Gew.% bei den Festsubstanzen. Für die Herstellung werden die magnetischen Kolloide der Polymerphase direkt zugemischt. Um eine feindisperse, gleichmäßige Verteilung der Partikel zu gewährleisten, ist ein kurzzeitiges Vermischen der wäßrigen Dispersion mittels eines hochtourigen Dispergierwerkzeuges (Ultra-Turrax) mit anschließender Ultraschallbehandlung förderlich.

Die zur Herstellung der Magnetpartikel benötigte Polymerphase besteht in der Regel aus 2.5-10 Gew% PVAL - Lösung. Wie aus der DE-OS 4127657 bekannt ist, wird die Porosität der Polymerteilchen letztlich durch die Knäueldichte bestimmt, die ihrerseits durch die mittlere Molmasse des Polymeren und die Konzentration festgelegt ist. Zunehmende Molmasse und/oder verringerte Polymerkonzentration bedeutet geringere Knäueldichte und damit zunehmende Porosität. Da die Praktikabilität eines Testverfahrens besonders im Rahmen routinemäßiger diagnostischer oder analytischer Verfahren auch von der Quantität der pro Trägermenge absorbierten Substanzen abhängt, spielt die Porosität als hierfür mitentscheidender Parameter eine wesentliche Rolle bei der Magnetpartikelherstellung. Bei den erfindungsgemäßen Mitteln kommen daher vorzugsweise Polymerkonzentrationen von 2.5-5 Gew% und Molmassen von >50..000 zum Einsatz. Auf diese Weise hergestellte Polymerpartikel weisen eine hohe Porosität und eine entsprechend hohe Bindungskapazität sowohl in Bezug auf die an die Matrix gekoppelten Liganden, als auch für die von den Liganden gebundenen Ligaten bzw. Biomoleküle auf. Ein weiterer Faktor, der in die Porosität und somit auch in die Funktionalität der Magnetpartikel einfließt, ist die Wahl des Vernetzers sowie dessen Konzentration. Im Hinblick auf hohe Beladungskapazitäten werden die Vernetzerkonzentrationen so gewählt, daß eine entsprechende Porosität in Verbindung mit ausreichender Formstabilität gewährleistet ist. Als Vernetzer kommen prinzipiell alle wasserlöslichen mit den Hydroxyl-Gruppen des PVAL reagierende bifunktionelle Verbindungen wie z.B. Aldehyde, Säurechloride oder Divinylsulfon in Frage. Vorzugsweise wird Glutaraldehyd unter Säurekatalyse als Vernetzer verwendet, da diese Substanz bereits innerhalb weniger Minuten mit dem Polymeren unter Bildung festvernetzter Partikel abreagiert. Bei den übrigen Substanzen sind ein bis zwei Stunden Reaktionszeit erforderlich. Die Verwendung von Glutaraldehyd bietet darüberhinaus überraschenderweise die Möglichkeit, durch simultane Zugabe eines wasserlöslichen Diamins, z.B. Äthylendiamin oder Hexamethylendiamin, die Vernetzereinheit entsprechend um die Länge der Diaminkette zu verlängern und dadurch die Porosität der Polymermatrix zu erhöhen. Die Vernetzerkonzentrationen liegen, bezogen auf die wäßrige Polymerphase, in der Regel zwischen 0.2 und 1 Vol% und für Glutaraldehyd zwischen 2 und 7 Vol%. Glutaraldehyd wird durchweg in Form einer 6-25%igen wäßrigen Lösung eingesetzt. Bei den Diaminen werden in der Regel zwischen 10 und 50 Mol%, bezogen auf die Glutaraldehyd Menge, eingesetzt.

Für die Herstellung der Magnetpartikel wird im allgemeinen zunächst die 20-25fache Volumenmenge einer organischen Phase, vorzugsweise handelsübliches Pflanzenöl, vorgegeben, in der anschließend die Polymer-Magnet-Kolloid-Mischung unter Rühren suspendiert wird. Die Zugabe der Säure im Falle der Glutaraldehyd-Vernetzung wird dabei von der Stabilität des Magnet-Kolloids bestimmt. Manche Magnet-Kolloide neigen dazu, bei Säurezugabe zu agglomerieren. Dies kann überraschenderweise dadurch umgangen werden, daß die Säure erst während oder am Ende des Suspensionsvorganges zugesetzt wird. Da die Magnet-Kolloide zu diesem Zeitpunkt bereits in der Polymermatrix feindispers veteilt sind, kann ein Agglomerieren gänzlich vermieden werden. Bei den säurestabilen Magnet-Kolloiden kann die Säure vor dem Suspensionsprozess auch direkt zu der Polymer-Phase zugegeben werden. Die Zugabe des Vernetzers erfolgt jeweils während des Suspensionsvorganges.

Neben den bisher beschriebenen Teilchengröße und - geometrie bestimmenden Parametern kann überraschenderweise gezeigt werden, daß die Säurekonzentration einen entscheidenden Einfluß auf die Suspendierbarkeit der Magnet-Teilchen hat. Gute Suspendierbarkeit bedeutet, daß die Teilchen völlig isoliert voneinander sind und in wäßrigen Lösungen keinerlei Agglomerate bilden. Diese Eigenschaft, die Voraussetzung für eine lange Verweilzeit im suspendierten Zustand ist, wird durch Säurekonzentrationen von 15-50 Vol%, bezogen auf die Polymerphase, erzielt. Es wird vorzugsweise 1-3N HCl verwendet. Die Verweilzeiten in der Suspension sind dementsprechend bei den PVAL-Partiklen sehr hoch, sie liegen zwischen 12 und 48 Stunden gegenüber 4 Stunden bei den Polystyrol-Beads aus der U.S. Patentschrift 4,654,267.

Den so gewonnenen Magnetpartikeln, deren besondere Vorzüge in den durch die vielfältigen Verfahrensparameter gestaltbaren und adaptierbaren Eigenschaften wie Porosität, Korngröße, magnetisches Verhalten und chemische Funktionalität begründet sind, eröffnen sich eine Vielzahl von Anwendungen, die in ihrer Gesamtheit den bisher beschriebenen Magnetträgern nicht zugänglich sind.

Aufgrund der hohen chemischen Funktionalität des Basispolymeren PVAL können bei den erfindungsgemäßen Mitteln sämtliche bei den herkömmlichen Affinitätschromatographie-Medien bekannten Aktivierungs- und Kopplungsverfahren angewendet werden. Beispiele für solche Aktivierungsagenzien sind: BrCN, 2-Fluor-1-methylpyridinium-toluol-4-sulfonat, 1,1'-Carbonyl-diimidazol, Epichlorhydrin, Hexamethylendiisocyanat oder 1-Cyano-4-dimethylamino-pyridinium-tetrafluorborat. Die entsprechenden Kopplungsmethoden für Bioliganden bzw. Biomoleküle sind allgemeiner Stand der Technik und u.a. in Methods in Enzymology, Vol. 135, K. Mosbach Hrsg., 1987, beschrieben. Die Kopplungsverfahren in dem vorgenannten U.S. Patent 4,654,267 sind demgegenüber auf die Aktivierung und Kopplung von carboxyl-Gruppen beschränkt.

Die vorhandenen Hydroxyl-Gruppen des hier beschriebenen erfindungsgemäßen Basispolymeren bieten darüberhinaus überraschenderweise die Möglichkeit, Vinylmonomere auf die Polymermatrix aufzupfropfen. Durch diesen Pfropfprozess können zusätzliche, funktionelle Molekülketten (Spacermoleküle) eingeführt werden. Die Kopplung der Biomoleküle an solche Spacermoleküle fördern allgemein die Erhaltung der nativen Struktur und damit die biologische Aktivität der gekoppelten Biomoleküle. Dadurch, daß das Biomolekül nicht mehr direkt mit der Matrixoberfläche in Kontakt kommt, werden mögliche Konformationsänderungen innerhalb des Biomoleküls unterbunden. Die Pfropfung mit Vinylmonomeren geschieht unter katalytischer Wirkung von Cer(IV)-Salzen, z.B. Cer(IV)-ammoniumnitrat oder Cer(IV)-ammoniumsulfat, die als Redoxinitiatoren für die Radikalpolymerisation fungieren. Die Cer(IV)-Salze werden bevorzugt als 0.03-0.1 molare Lösungen in 0.5- 1 N Schwefelsäure oder Salpetersäure eingesetzt. Als Vinylmonomere kommen solche Substanzen zum Einsatz, die über funktionelle oder reaktive Gruppen z.B. in Form von HO-, HOOC-, NH2-, NCO-, CHO- oder Oxiran-Gruppen verfügen. Bezüglich der Einzelheiten des an sich bekannten Pfropfverfahrens wird auf die in den Offenlegungsschriften DE-OS 2157902 und DE-OS 3811042 beschriebenen Verfahren verwiesen. Die hier vorgestellten erfindungsgemäßen Polymermatrizes unterscheiden sich jedoch hinsichtlich ihrer physikalischen und chemischen Struktur, Eigenschaften und Anwendtbarkeit grundsätzlich von der vorgenannten Pfropfmatrizes. Ein weiterer Unterschied zu den vorgenannten Pfropfverfahren besteht darin, daß bei den erfindungsgemäßen Mitteln nicht unter Sauerstoffausschluß gearbeitet werden muß, sondern daß die Gegenwart eines mit Wasser nicht mischbaren organischen Lösungsmittels wie z.B. Hexan, Heptan, Cyclohexan oder Petroläther ausreicht, um hohe Pfropfausbeuten zu realisieren. Die Pfropfzeiten lassen sich darüberhinaus gegenüber den vorgenannten Verfahren bis zu 90% verringern. Die Mengen der eingesetzten Vinylmonomeren bewegen sich zwischen 10 und 50 Vol%, bezogen auf die Magnetpartikel-Suspension.

Aufgrund der vielfältigen Aktivierungs- und Modifikationsmöglichkeiten der PVAL-Magnetpartikel kann eine praktisch unbegrenzte Anzahl von Biomolekülen an die Matrix gekoppelt werden. Es ergeben sich so weitreichende Einsatzmöglichkeiten, die von der medizinischen Diagnostik bis hin zur molekularbiologischen Analytik reichen. Eine wichtige Anwendung innerhalb der Biowissenschaften stellen die Auftrennungen nach dem Affinitätsprinzip dar. Die hierfür normalerweise verwendete Säulentechnik ist jedoch mit erheblichen experimentellen Aufwand verbunden, so dass vor allem für kleine, routinemäßige Auftrennungen praktische Alternativen wünschenwert sind. Die erfindungsgemäßen Mittel bieten solche Alternativen, da der Zeit- und experimentelle Aufwand nur einen Bruchteil gegenüber dem herkömmlicher Techniken erfordert. Als Liganden können grundsätzlich sämtliche heute in der Affinitätschromatographie verwendeten Liganden gekoppelt werden. Beispiele hierfür, die auch aus praktischer Sicht interessante Perspektiven eröffnen, sind: Protein A, Protein G, Heparin, Antikörper, Serum Albumin, Gelatine, Lysin, Concavalin A, Oligosaccharide, Oligonucleotide oder Enzyme. Die speziellen Auftrennungen, die sich mit solchen Affinitätsmatrizes durchführen lassen, sind allgemeiner Stand der Technik. Bezüglich der Einzelheiten dieser an sich bekannten Verfahren wird auf die Ausführungen im J. of Chromatography, Vol. 510, 1990, verwiesen. Neben der experimentellen Vereinfachung liegt der besondere Vorteil der Magnetpartikel-Technologie jedoch in einer deutlichen Verkürzung der Auftrennzeiten. Dies liegt darin begründet, daß die Magnetpartikel-Suspension eine quasi-homogene Phase darstellt, die eine Umsatzkinetik analog der einer homogenen Lösung ermöglicht. Auf diese Weise lassen sich Auftrennungen ohne größeren Aufwand je nach Größe des Ansatzes innerhalb von 2-5 Minuten durchführen.

Ein weiterer, interessanter Bereich, der mit der Magnetpartikel-Technologie abgedeckt werden kann, ist der Bereich der Diagnostik, im besonderen der Bereich des Immunoassays. Das grundlegende Prinzip besteht darin, spezifische Substanzen quantitativ zu erfassen. Die Qualität des Nachweises ist dabei unmittelbar an die spezifische Isolierung der betreffenden Substanz, sei es durch chromatographische Verfahren oder durch Bindung an einen polymeren Festkörper, geknüpft. In der Praxis geschieht diese spezifische Bindung in der Regel über einen immobilisierten Antikörper, der dann photometrisch oder nephelometrisch analysiert wird. Die neu entwickelten PVAL-Medien bieten hier nun eine hervorragende Basis, für Immunoassays eingesetzt zu werden. Dazu werden in der bekannten Art und Weise Antikörper gegen bestimmte, für die Diagnose relevante Antigene an die Magnetpartikel chemisch gebunden. Beispiele solcher Antikörper sind: anti-Insulin, anti-Thyroxin, Antikörper gegen das Thyroid-stimulierende Hormon (TSH), Antikörper gegen das Thyroid bindende Globulin, anti-Cortison, anti-Ferritin, anti-Chorionic Gonadotropin, anti-Carcinogen-Embryonales-Antigen (CEA), anti-Progesteron, anti-Testosteron, anti-Estradiol, anti-Prolactin, anti-Human-Growth-Hormon, anti-Digoxin, anti-β2-Microglobulin, anti-α2-Macroglobulin, anti-Vitamin B12, anti-Faktor VIII oder anti-AFP. Die Inkubationzeiten der Antikörper-gekoppelten Magnetpartikel mit den Substanzgemischen beträgt in der Regel 2-5 Minuten. Nach magnetischer Abtrennung werden die isolierten Antikörper-Antigen Komplexe photometrisch mit Hilfe der bekannten Analysenmethoden quantitativ detektiert. Durch die Verwendung der Magnetpartikel-Technologie lassen sich die Inkubationszeiten um den Faktor 10-100 gegenüber der herkömmlichen Mikrotiterplatten- oder Säulentrenntechnik, wie sie in der DE-OS 4126436 beschrieben ist, verkürzen. Außer Antikörper können auch andere Substanzen an die Magnetpartikel gekoppelt werden und zur Detektierung bestimmter Substanzen genutzt werden. Eine solche Substanz ist 3-Aminophenylboronsäure, die an die PVAL-Matrix gekoppelt, zur Detektion des Blutzuckergehaltes verwendet wird. Zur Immobilisierung des Liganden wird der PVAL-Träger im ersten Schritt mit Diisocyanaten aktiviert. Anschließend erfolgt die Umsetzung mit dem Liganden. Für die Umsetzung werden in Regel 15-30 mg 3-Aminophenylboronsäure pro 100 mg Magnetphase eingesetzt. Die Analyse des Blutzuckergehaltes geschieht über das im Blut vorhandene glykierte Hämoglobin, das sich spezifisch an die Boronsäure-Liganden bindet. Durch anschließende Elution der gebundenen glykierten Fraktion von der Matrix wird diese quantitativ mittels photometrischer Methoden analysiert. Der besondere Vorzug gegenüber den früheren Testverfahren ist der geringere arbeitstechnische Aufwand. Die Methode kann daher besonders für routinemäßige Analysen eingesetzt werden,

Die in den letzten Jahren im Zuge neuer therapeutischer- und diagnostischer Maßnahmen sehr in den Vordergrund gerückten molekularbiologischen Analysen stellen ein weiteres Anwendungsfeld für die PVAL-Magnetpartikel dar.

Bei vielen analytischen Verfahren innerhalb der Molekularbiologie wird die hohe Affinität zwischen Streptavidin/Avidin und Biotin genutzt. Durch Ankopplung von Streptavidin bzw. Avidin an polymere Festphasen können biotinylierte Biomoleküle jeglicher Art wie z.B. DNA-Fragmente, Oligonukleotide, Proteine, Antikörper oder Antigene isoliert werden. Die Verwendung der PVAL-Matrix bietet hier aufgrund der einfachen Kopplungstechnik in Verbindung mit der hohen Suspendierfähigkeit die Möglichkeit, solche Trennungen in einfacher Weise durchzuführen. Praktische Anwendungsbereiche, wo die Magnet-Bead-Technik vorzugsweise eingesetzt werden kann, sind z.B.: DNA-Festphasen-Sequenzierungen, DNA-Synthesen oder Polymerase-Ketten-Reaktion (PCR). Die PVAL-Magnetpartikel bieten ferner über die Kopplung von Antikörpern, die gegen bestimmte Zellmarker gerichtet sind, z.B. anti-CD4, anti-CD15, anti-CD35, anti-CD8, Zellseparationen und - Markierungen (Labelling) durchzuführen.Bezüglich der Einzelheiten wird auf die bekannte Literatur verwiesen: Haukanes und Kram, Biotechnology, Vol .11, 1993, 60.

Die Erfindung wird in den nachfolgenden Beispielen näher erläutert.

### Beispiel 1

Ein Magnetit-Kolloid wird analog der Vorschrift von Kondo et al., Appl. Microbiol Biotechnologie, Vol. 41, 1994, 99-105, hergestellt. 10 ml des Kolloids werden in eine Mischung, bestehend aus 80 ml 10% PVAL-Lösung (mittlere Molmasse 48.000), 5 ml 2.5% Polyvinylpyrrolidon Lösung, 20 ml 2 N HCl und 0.4 ml 3.5% Na-Dodecylsulfat, dispergiert. Nach einminütiger Behandlung im Ultraschallbad (100 W) wird die Mischung bei 20°C in 2 Liter eines herkömmlichen Pflanzenöls, das 2% Pluronic 6100, 0.8% Pluronic 6200, 0.4 % Dehymuls FCE und 0.6% Dehymuls HRE 7 enthält unter Rühren suspendiert. Die Rührgeschwindigkeit beträgt 2000 U/Min. Nach 10 Sek. werden 6.4 ml 12%ige Glutaraldehyd-Lösung zugegeben. Es wird noch 20 Sek. weitergerührt. Danach wird die Suspension bei 5000 x g 30 Sek. zentrifugiert und die Ölphase abdekantiert. Die verbleibende Suspension wird einmal mit je ca. 300 ml n-Hexan und Methyläthylketon nachgewaschen. Die gewonnene magnetische Suspension wird in ca. 400 ml Wasser/Methanol 1:1 (v/v) dispergiert und wiederum abzentrifugiert. Danach erfolgt 10maliges Waschen der Magnetfraktion durch Dispergieren in ca. 400 ml Wasser mit jeweils dazwischengeschaltetem Zentrifugationschritt. Es werden Magnetpartikel mit einer Größenverteilung von 2-4 µm und einem Eisengehalt von 7% gewonnen.

(Alle %-Angaben hier und im folgenden sind in Vol.% , sofern es sich um eine flüssige Substanz und in Gew.% , sofern es sich um eine Festsubstanz handelt).

### Beispiel 2

5 ml Magnet-Kolloid gemäß Beispiel 1 werden in 40 ml PVAL-Phase gemäß Beispiel 1 dispergiert und anschließend in 880 ml handelsüblichem Pflanzenöl, in dem 1.5% Pluronic 8100, 0.4 % Pluronic 6200 und 0.4% Dehymuls FCE gelöst sind, unter Rühren suspendiert (Rührgeschwindigkeit 2000 U/Min.). Sodann werden 4% 25%ige Glutaraldehyd-Lösung zugegeben; die Suspension wird für weitere 10 Sek. gerührt. Nach 10 Minuten wird die Suspension abzentrifugiert und gemäß Beispiel 1 mit Methanol/Wasser, n-Hexan und Methyläthylketon gewaschen. Es werden Magnetpartikel mit einer Größe von 1-3 µm gewonnen. Der Eisenoxid-Anteil beträgt 7.3 %.

### Beispiel 3

4 ml Ferrofluidics EMG 807 werden in 100 ml 5% PVAL-Lösung (mittlere Molmasse 224.000) dispergiert. Die Dispersion wird 5 Min. im Ultraschallbad behandelt und anschließend in 2300 ml Pflanzenöl, das 2% Arlacel 83, 0.8% Tween 85 und 0.4% Dehymuls FCE enthält, unter Rühren (Rührgeschwindigkeit 1800 U/Min.) suspendiert. Nach 5 Sek. werden 25 ml 2.5 N HCl zugegeben und nach weiteren 5 Sek. 7 ml 12%ige Glutaraldehyd-Lösung. Es wird noch 10 Sek. weitergerührt. Die Suspension wird nach 10 Min., wie unter Beispiel 1 beschrieben, abzentrifugiert und gewaschen. Es entstehen Magnetpartikel mit einer Korngröße von 2-5 µm und einem Eisenoxidgehalt von 24.6%.

### Beispiel 4

180 mg Bayferrox Eisenoxid-Pigment PR 5044 N werden in 20 ml 7%iger PVAL-Lösung (mittlere Molmasse 88.000), die 0.01% Polystyrolsulfonsäure und 0.05 % Polyäthylenglykol 1000 enthält, vermischt und eine Min. mit einem Dispergierwerkzeug (Ultra-Turrax ) bei 20.000 U/Min. dispergiert. Es folgt eine zweimal 2minütige Behandlung im Ultraschallbad. Die Mischung wird anschließend unter Rühren (Rührgeschwindigkeit 2000) in 460 ml Pflanzenöl, das 1.9% Arlacel 83, 0.4% Tween 20, 0.3% Dehymuls FCE und 1% Dehymuls HRE 7 enthält, dispergiert. Nach 10 Sek. wird 0.8 ml 25%ige Glutaraldehyd-Lösuing zugefügt und nach weiteren 5 Sek. 8 ml 1 N HCl. Die Suspension wird noch 10 Sek. weitergerührt. Nach 10 Min. erfolgt die Isolierung und Aufarbeitung der Fraktion gemäß Beispiel 1. Es fallen Magnetpartikel mit einer Korngrößenverteilung von 2-4 µm und einem Eisenoxidgehalt von 12.3% an.

### Beispiel 5

50 mg Bayferrox 318 M werden in 10 ml einer Mischung, bestehend aus 5% PVAL (mittlere Molmasse 224.000), 0.01% Na-Dodecylsulfat und 0.1% Polyäthylenglykol 1000 mittels Ultra-Turrax dispergiert (20.000 U/Min.). Anschließend wird die Polymerphase in 250 ml Pflanzenöl, das 2.2% Span 80, 0.4% Dehymuls FCE und 0.4% Pluronic 6200 enthält, unter Rühren suspendiert (Rührgeschwindigkeit 1800). Nach 5 Sek. werden 10 ml 1 N HCl zugefügt und nach weiteren 10 Sek. 0.6 ml 25%ige Glutaraldehyd-Lösung. Es wird 10 Sek. weitergerührt und die Suspension nach 5 Min. abzentrifugiert. Die gewonnene Fraktion wird gemäß Beispiel 1 gewaschen. Es bilden sich Magnetpartikel mit einer Korngrößenverteilung von 3-6 µm, deren Eisengehalt 10.2 % beträgt.

### Beispiel 6

In eine Mischung, bestehend aus 50 ml 4%iger PVAL-Lösung (mittlere Molmasse 103.000), in der 0.1% Rinder Serum Albumin und 0.5% Polyäthylenglykol 1000 gelöst sind, werden 6.4 ml Magnet-Kolloid gemäß Beispiel 1 dispergiert. Die Dispersion wird 5 Min im Ultraschallbad beschallt und anschließend in 1100 ml Pflanzenöl, das 1.8% Span 85, 0.8% Synperonic Pl 61, 0.8% Tetronic 901 und 0.4% Dehymuls FCE enthält, suspendiert (Rührgeschwindigkeit 2000). Nach 10 Sek. werden 4 ml 12%ige Glutaraldehyd-Lösung zugegeben und nach weiteren 5 Sek. 12.5 ml 2.5 N HCl. Die Suspension wird noch 10 Sek. weitergerührt. Nach 15 Min. erfolgt die Zentrifugation und weitere Aufarbeitung gemäß Beispiel 1. Es werden Magnetpartikel mit einer Korngrößenverteilung von 1-3 um und einem Eisenoxidgehalt von 8.3% erhalten.

### Beispiel 7

100 ml 3.5 %ige PVAL-Lösung (mittlere Molmasse 224.000), die 20% 3 N HCl enthält, werden mit 13 ml Ferrofluidics EMG 507 vermischt und 0.5 Min. im Ultraschallbad beschallt. Die Magnetit-Polymer-Phase wird sodann in 2.3 Liter Pflanzenöl, das 1.8% Pluronic 6100, 0.2% Pluronic 6200, 0.2% Hypermer A60 und 1.8% Dehymuls HRE 7 enthält unter Rühren suspendiert (Rührgeschwindigkeit 2000). Nach 10 Sek. werden 8 ml 12%ige Glutaraldehyd-Lösung zugegeben und 15 Sek. weitergerührt. Nach 10 Min. wird die Suspension abzentrifugiert und gemäß Beispiel 1 gewaschen. Die gewonnenen Teilchen haben eine Korngrößenverteilung von 1-2 um und weisen einen Eisenoxidgehalt von 14.2% auf.

### Beispiel 8

100 ml 7.5% ige PVAL-Lösung (mittlere Molmasse 88.000), in der 0.05% Gelatine gelöst sind, werden mit 12.5 ml Ferrofluidics EMG 707 vermischt und 3 Min. im Ultraschallbad behandelt. Die Mischung wird anschließend in 2.5 Liter Pflanzenöl, das 1% Arlacel 83, 0.4% Pluronic 6100 und 0.2% Brij 52 und 0.4% Tween 60 enthält, unter Rühren suspendiert (Rührgeschwindigkeit 2000). Nach 10 Sek. werden 4 ml 12%ige Glutaraldehyd-Lösung und nach weiteren 5 Sek. 26.5 ml 1 N HCl zugefügt. Die Suspension wird noch 15 Sek. weitergerüht. Nach 15 Min. wird die Suspension abzentrifugiert und gemäß Beispiel 1 gewaschen. Es fallen Magnetteilchen mit einer Korngrößenverteilung von 2-4 µm und einem Eisenoxidgehalt von 26.0% an.

### Beispiel 9

10 ml 7.5 % ige PVAL-Lösung (mittlere Molmasse 103.000) werden mit 0.5 N NaOH auf pH 9.5 eingestellt und 75 µl Divinylsulfon zupipettiert. In der wäßrigen Phase werden sodann 1.2 ml Magnet-Kolloid gemäß Beispiel 1 dispergiert. Nach 3minütiger Beschallung im Ultraschallbad wird die Mischung in 220 ml Pflanzenöl, in dem 2% Span 60, 0.4% Tween 80 und 0.4% Dehymuls FCE gelöst sind, unter Rühren suspendiert (Rührgeschwindigkeit 2000). Es wird noch 30 Sek. weitergerührt. Danach wird die Suspension 60 Min. bei Raumtemperatur stehengelassen und anschließend gemäß Beispiel 1 aufgearbeitet. Es werden Partikel mit einer Korngrößenverteilung von 4-8 µm gewonnen. Der Eisenoxidgehalt beträgt 7.7%.

### Beispiel 10

100 ml 3.5%ige PVAL-Lösung (mittlere Molmasse 224.000), in der 40% 1 N HCl und 0.015 % Na-Dodecylsulfat gelöst sind, werden mit 5 ml Ferrofluidics EMG 707 versetzt und eine Min. im Ultraschallbad beschallt. Die Polymerphase wird sodann in 2.3 Liter Pflanzenöl, in dem 1%Arlacel 83, 1% Pluronic 6100, 0.8% Tween 80 und 2% Dehymuls HRE gelöst sind, unter Rühren (Rührgeschwindigkeit 2000) 10 Sek. suspendiert. Nach 10 Sek. werden 6 ml 25%ige Glutaraldehyd-Lösung zugegeben; es wird für weitere 10 Sek. gerührt. Nach 10 Min. wird die Suspension gemäß Beispiel 1 abzentrifugiert und gewaschen. Es bilden sich Magnetpartikel mit einer Korngrößenverteilung von 2-4 µm, die einen Eisenoxidgehalt von 24% aufweisen

### Beispiel 11

14.5 ml Magnetit-Kolloid gemäß Beispiel 1 werden in 100 ml Polymerphase, die 4% PVAL (mittlere Molmasse 224.000) und 0.1% Rinder Serum Albumin enthält, dispergiert und eine Min. im Ultraschallbad behandelt. Danach wird die Dispersion zunächst für 15 Sek. in 2.5 Liter Pflanzenöl, in dem 3.8% Pluronic 3100, 0.8% Pluronic 6200 und 1.5% Tetronic 304gelöst sind, unter Rühren suspendiert (Rührgeschwindigkeit 2000). Es werden sodann 7.5 ml 12%ige Glutaraldehyd-Lösung und nach weiteren 10 Sek. 25 ml 3 N HCl zugegeben ; es wird noch für 10 Sek. weitergerührt. Nach 10 Min. wird die Suspension gemäß Beispiel 1 aufgearbeitet. Es werden Magnetpartikel mit einer Korngrößenverteilung von 1-2 um und einem Eisenoxidgehalt von 9.6 % gewonnen.

### Beispiel 12

In 1200 ml Pflanzenöl, das2.2% Arlacel 80, 0.8% Span 85 und 0.8% Triton CF 10 enthält, werden 50 ml Polymerphase bestehend aus 5% PVAL (mittlere Molmasse 224.000), 0.5% Polyäthylenglykol 3350 und 12% Ferrofluidics EMG 707 unter Rühren (Rührgeschwindigkeit 1800) 10 Sek. suspendiert. Es folgt in je 5 Sek. Abständen die Zugabe von 4 ml 25%ige Glutaraldehyd-Lösung und 25 ml 1 N HCl. Rühren wird für 15 Sek. fortgesetzt. Nach 10 Min. wird abzentrifugiert und gewaschen gemäß Beispiel 1. Man erhält Magnetpartikel, die eine Korngrößenverteilung von 1-2 µm aufweisen und einen Eisenoxidgehalt von 18.3% aufweisen.

### Beispiel 13

In 100 ml 5%ige PVAL-Lösung (mittlere Molmasse 203.000), in der 0.05% Polystyrolsulfonsäure und 0.1% Polyvinylpyrrolidon gelöst sind, werden mit 12 ml Magnetit-Kolloid gemäß Beispiel 1 dispergiert und 2 Min. im Ultraschallbad beschallt. Es erfolgt die Suspension in 2.2 Liter Pflanzenöl, dessen Zusammensetzung analog Beispiel 12 ist. Nach 10 Sek. werden in Abständen von je 10 Sek. 8 ml 12%ige Glutaraldehyd-Lösung und 20 ml 2.5 N HCl zugegeben. Nach entsprechender Aufarbeitung analog Beispiel 1 erhält man 2-4 µm große Magnetpartikel mit einem Eisenoxidgehalt von 7.5%

### Beispiel 14

6.5 ml Ferrofluidics EMG 807 werden in 100 ml Polymer-Phase, bestehend aus 10% PVAL (mittlere Molmasse 88.000), 0.05% Cellulose-acetat-butyrat und 0.1% Polyvinylpyrrolidon, dispergiert und 3 Min. im Ultraschallbad beschallt. Die Dispersion wird anschließend in 2300 ml Pflanzenöl , das 1.8% Synperonic L61, 0.2% Tetronic 1101 und 1% Dehymuls FCE enthält, unter Rühren (Rührgeschwindigkeit 2000 U/Min.) suspendiert. Nach 10 Sek. werden in je 10 sekündigen Intervallen je 8 ml 12%ige Glutaraldehyd-Lösung, die 20 Mol% Äthylendiamin enthält, und 23 ml 2.5 N HCl zugegeben. Es wird 10 Sek. weitergerührt und die Suspension nach 10 Minuten analog Beispiel 1 aufgearbeitet. Man erhält Magnetpartikel mit einer Korngrößenverteilung von 1-3 µm und einem Eisenoxidgehalt von 10.4 %.

### Beispiel 15

300 mg, der nach Beispiel 1 hergestellten Polymerpartikel, werden in 10 ml Wasser suspendiert, mit 10 ml 3.5 M NaOH und 15 ml Epichlohydrin versetzt und 2 h bei 55°C unter intensivem Rühren umgesetzt. Danach werden die Magnetpärtikel mittels eines Neodym-Eisen-Bor-Magneten abgetrennt. Das Produkt wird in ca.10 ml Wasser suspendiert und nochmals magnetisch abgetrennt. Dieser Wasch/Abtrennvorgang wird 10mal wiederholt, gefolgt von einmaligem Waschen mit Aceton. 30 mg der so aktivierten Magnetpartikel werden sodann mit 2 ml 0.1 M Borat-Puffer, pH 11.4, der 10% Hexamethylendiamin enthält, bei 50°C 2 h umgesetzt. Es wird 10mal mit Wasser nachgewaschen. Das gewonnene Produkt wird anschließend mit 2 ml 0.1 M K-Phosphat-Puffer, pH 7.0, in dem 12.5% Glutaraldehyd gelöst ist, für 2 h bei 30°C zur Reaktion gebracht. Anschließend wird über einen Zeitraum von 30 Min. zunächst 10mal mit Wasser und dann 2mal mit 0.1 M K-Phosphat-Puffer, pH 7.5, nachgewaschen. Durch Zugabe von 1 ml 0.1 M K-Phosphat-Puffer, pH 7.5, in dem 0.3 mg Streptavidin gelöst sind, werden nach 12 stündiger Inkubation bei 4 °C 0.11 mg Streptavidin an die Matrix gebunden. An die Matrix lassen sich nach den bekannten Methoden biotinylierte DNA-Fragmente binden, die zur DNA-Sequenzierungen eingesetzt werden.

### Beispiel 16

30 mg der gemäß Beispiel 15 gewonenen Epichlorhydrin/Hexamethylendiamin/Glutaraldehyd-aktivierten Magnetpartikel werden mit 2 ml 0.1 M K-Phosphat-Puffer, pH 7.5, in dem 0.3 mg anti-Insulin Antikörper gelöst sind, 24 h bei 4°C umgesetzt. Es werden 0.2.8 mg Antikörper gebunden.

### Beispiel 17

60 mg Magnetpartikel gemäß Beispiel 3 werden durch sukzessive Zugabe von Aceton/Wasser-Mischungen 1:3, 1:1, 3:1 (v/v) und schließlich absolutem Aceton entwässert. Danach wird die Suspension in 2 ml absolutem Dimethylsulfoxid, das 0.1% Zinn-octoat enthält, dispergiert und durch Zugabe von 0.5 ml Hexamethylendiisocyanat für 30 Min. bei 45°C aktiviert. Danach wird je 5mal abwechselnd mit einigen ml Dimethylsulfoxid und Aceton gewaschen. 30 mg der aktivierten Fraktion werden mit 1 ml absolutem Dimethylsulfoxid, in dem 20% Polyäthylenglykol 400 und 0.05% DABCO gelöst sind , 4 h bei 30°C umgesetzt. Es wird einmal mit Dimethylsulfoxid und 5mal mit Wasser gewaschen und die Fraktion wiederum mit den obigen Aceton/Wasser-Mischungen entwässert. Die Polyäthylenglykol-gekoppelte Fraktion wird sodann mit je 1 ml Dimethylsulfoxid, in dem 6 mMol 4-Dimethylamino-pyridin und 5 mMol 2-Fluor-methyl-pyridinium-toluol-sulfonat gelöst ist, 45 Min. bei Raumtemperatur umgesetzt. Es folgt 5maliges abwechselndes Waschen mit Dimethylsulfoxid und Aceton. Das aktivierte Produkt wird anschließend bei 4°C durch Inkubation mit einer anti-Thyroxin Antikörper-Lösung (0.25 mg Antikörper/ml 0.05 M K-Phosphat-Puffer, pH 7.5) gekoppelt. Es werden 0.23 µg Antikörper gekoppelt. Nach mehrfachem Waschen mit dem Kopplungspuffer werden die restlichen aktiven Gruppen durch 4stündige Inkubation mit 2 ml 20 mMol Mercaptoäthanol enthaltendem 0.1 M Tris-HCl Puffer, pH 8.5, desaktiviert. Danach werden Magnetpartikel mit PBS Puffer, pH 7.2, gewaschen. Die gekoppelten Magnetpartikel werden nach den bekannten Verfahren für die Thyroxin-Bestimmung eingesetzt.

### Beispiel 18

30 mg der gemäß Beispiel 17 mittels Hexamethylendiisocyanat aktivierten Fraktion werden 5 h mit 2 ml 0.3 KOH/MeOH-Lösung 1:1 (v/v) versetzt. Nach 5stündiger Reaktion bei Raumtemperatur wird mehrfach mit Wasser gewaschen. Die Aminogruppen enthaltende Fraktion wird dann gemäß Beispiel 15 mit Glutaraldehyd aktiviert. Es folgt merfaches Wasschen mit Wasser über einen Zeitraum von 30 Min.. Durch 20stündige Inkubation bei 4°C mit 1 ml 0.1 M K-Phosphat-Puffer, pH 7.5, in dem 0.35 mg anti-Maus IgG gelöst sind, werden 0.28 mg IgG gebunden. Das gekoppelte Produkt wird nach den bekannten Verfahren für die Zellseparation eingesetzt.

### Beispiel 19

30 mg Magnetpartikel gemäß Beispiel 10 werden bei 0°C mit 4 ml Wasser, in dem 100 mg BrCN gelöst sind, versetzt. Durch Zugabe von 2 N NaOH wird der pH auf 11.5 eingestellt. Die Reaktion wird nach 5 Min. durch magnetische Abtrennung der Magnetfraktion beendet. Es folgt 4maliges Waschen mit Eiswasser. Danach wird je einmal mit 0.01 N HCl und 0.1 M Bicarbonat-Puffer, pH 8.2, nachgewaschen. 1 ml 0.1 M Bicarbonat-Puffer, pH 8.2, in dem 0.2 mg anti-CD4 Antikörper gelöst sind, wird mit den aktivierten Magnetbeads. für 12 h bei 4°C inkubiert. Es wird mehrfach mit PBS-Puffer, pH 7.2, der 0.5 M NaCl und 0.1 % Triton X100 enthält, nachgewaschen. Man erhält einen Träger an den 0.18 mg Antikörper gebunden sind. Die gewonnen Träger werden zur Isolierung von T-Helferzellen verwendet.

### Beispiel 20

30 mg Magnetpartikel gemäß Beispiel 5 werden analog Beispiel 19 mit BrCN aktiviert. Ankopplung von Heparin (Molmasse 6000) erfolgt durch 12stündige Inkubation von 1 ml Bicarbonat-Puffer, pH 8.2, der 0.5 mg gelöstes Heparin enthält. Danach wird für 2 h bei Raumtemperatur mit 0.1 M Äthanolamin-Lösung, pH 8.0, inkubiert und anschließend 4mal mit 0.1 M Acetat-Puffer, pH 4, nachgewaschen. Die gewonnene Fraktion wird nach den bekannten Verfahren zur Trennung von Antithrombin III verwendet.

### Beispiel 21

30 mg Magnetpartikel gemäß Beispiel 4 werden durch sukzessive Zugabe von Aceton/Wasser Mischungen, wie oben beschrieben, entwässert. Je 1 ml Dimethylsulfoxid, in dem 6 mMol 4-Dimethylamino-pyridin und 5 mMol 2-Fluor-methylpyridinium-toluol-sulfonat gelöst sind, wird mit den Magnetbeads für 45 Min. bei Raumtemperatur inkubiert. Danach wird je 5mal abwechselnd mit Aceton und Dimethylsulfoxid sowie einmal mit 0.05 M K-Phosphat-Puffer, pH 7.5, gewaschen. Anschließend wird 1 ml 0.05 K-Phosphat-Puffer, pH 7.5, der 0.3 mg Protein A enthält zugesetzt. Die Kopplung erfolgt über einen Zeitraum von 12 h bei Raumtemperatur. Es folgt mehrfaches Waschen mit PBS-Puffer, in dem 0.5% NaCl und 0.1%Triton X100 gelöst sind. Es werden 0.27 mg Protein gebunden. Die Magnetpartikel Fraktion wird zur Abtrennung von IgG -Subklassen analog den bekannten verfahren benutzt.

### Beispiel 22

30 mg Magnetpartikel Fraktion gemäß Beispiel 12 werden einmal mit 2 ml 0.5 M Carbonat-Puffer, pH 11 gewaschen. Die anschließende Aktivierung mit 100 µl Divinylsulfon erfolgt unter Zugabe von 1 ml Wasch-Puffer über einen Zeitraum von 70 Min. bei Raumtemperatur. Es folgt mehfaches Waschen mit Wasser über eine Zeitraum von 30 Min. 1 ml Carbonat-Puffer, pH 10, der 10% Lactose enthält, wird für 20 h bei Raumtemperatur mit der Magnetbead Fraktion inkubiert. Es werden 0.68 mg Lactose gebunden. Der Träger wird zur Aufreinigung von Lektinen aus Mistelextrakten nach den bekannten Verfahren verwendet.

### Beispiel 23

30 mg Magnetpartikel gemäß Beispiel 7, die analog Beispiel 15 mit Epichlorhydrin aktiviert wurden, werden 16 h bei Raumtemperatur mit 0.3 M Adipinsäuredihydrazid in 0.2 M Carbonat-Puffer, pH 9, zum Hydrazid-Derivat umgesetzt. Es wird mehrfach mit 0.1 M Tris-HCl Puffer, pH 8.5, nachgewaschen. Restliche Oxiran-Gruppen werden durch 4stündige Inkubation mit 0.3 M Mercaptoäthanol-Lösung enthaltendem Waschpuffer-Lösung desaktiviert. Es wird mit 3 ml 0.1 M Na-Acetät-Puffer, pH 5.5, nachgewaschen. 1 ml Wasch-Puffer, in dem 0.3 mg anti-HGH und 10 mMol Na-m-Perjodat gelöst sind, werden unter Lichtabschluß für 40 Min. bei 4°C inkubiert. Es folgt 5maliges Waschen mit Na-Acetat-Puffer und 2maliges Waschen mit PBS Puffer. Es werden 0.21 mg Antikörper gebunden. Der gekoppelte Träger wird zur Bestimmung von HGH verwendet.

### Beispiel 24

30 mg Magnetpartikel gemäß Beispiel 9 werden in Wasser suspendiert und per Handmagnet abgetrennt. Die abgetrennte Fraktion wird in 0.5 ml 0.1 M Salpetersäure, die 0.1 M Cer(IV)-ammoniumnitrat enthält, für 15 Min. bei Raumtemperatur inkubiert. Danach werden die Magnetpartikel magnetisch abgetrennt und einmnal mit Wasser nachgewaschen. Die Fraktion wird sodann unter Zugabe von 0.5 ml Acrylsäure und 0.5 ml n-Hexan für 15 Min. bei 50 °C zur Reaktion gebracht. Es wird 10mal mit Wasser über einen Zeitraum von 30 Min. nachgewaschen. Die Pfropfausbeute beträgt 85 %. Die gepfropfte Fraktion wird mit 1ml 0.1 M MOPS-Puffer (3-Morpholino-propansulfonsäure), pH 7.5, der 5% N-Cyclohexyl-N-'(2-morpholinoäthyl)-carbodiimid-methyl-p-toluolsulfonat enthält, 30 Min. bei Raumtemperatur aktiviert. Es wird 5mal mit Eiswasser nachgewaschen und 0.3 mg anti-LDL Antikörper, gelöst in 1 ml MOPS-Puffer, pH 7.5, zugesetzt. Reaktionsdauer 24 h bei 4°C. Danach wird der Träger durch 4stündige Inkubation mit 5 ml 5mM Äthanolamin/0.1 M Tris-HCl-Puffer, pH 8.5, desaktiviert. Es werden 0.25 mg Antikörper gebunden. Die gekoppelte Matrix wird zur Entfernung von LDL (Low-density-Lipoprotein) aus biologischen Flüssigkeiten eingesetzt.

### Beispiel 25

30 mg Magnetpartikel gemäß Beispiel 3 werden analog Beispiel 24 mit Acrylsäure gepfropft. 100 µg Oligo(dT)20 werden entsprechend der Vorschrift in DNA, Vol. 4, 1988, 327 mit Äthylendiamin am 5'-Ende substituiert. Das NH2-modifizierte Oligonukleotid wird in 1 ml 0.1 M Imidazol-Puffer, pH 7.0, gelöst und mit der Magnetpartikel Fraktion für 20 h bei Raumtemperatur inkubiert. Danach wird mehrfach mit dem Imidazol-Puffer, der 0.1% Triton X100 enthält, gewaschen. Es werden 38 µg Oligonukleotid gebunden. Die gewonnenen Magnetpartikel werden zur Isolierung von mRNA nach den bekannten Verfahren verwendet.

### Beispiel 26

100 mg Magnetpartikel gemäß Beispiel 9 werden zunächst mit Hilfe der Aceton/Wasser-Mischungen gemäß obiger Vorschrift entwässert. Es folgt die Aktivierung mittels Hexamethylendiisocyanat gemäß Beispiel 17. 2 ml Dimethylsulfoxid, in dem 0.1% Zinn-Octoat und 30 mg m-Aminophenylboronsäure gelöst sind, werden mit den aktivierten Partikeln für 6 h bei 30°C inkubiert. Danach wird mehrfach mit Wasser nachgewaschen. Die Boronsäure gekoppelten Magnetpartikel werden zur Bestimmung des glykierten Hämoglobins im Blut nach den bekannten Verfahren eingesetzt.

### Beispiel 27

30 mg Magnetpartikel entsprechend Beispiel 14 werden mit 2 ml Wasser, in dem 50 mg Cibacron Blau F3G-A gelöst sind, versetzt und für 30 Min. auf 60°C erhitzt. Danach wird 1 ml 25% NaCl-Lösung zugesetzt und eine weitere h auf 75°C erhitzt. Nach Zugabe von 1 ml 10%iger Na-Carbonat-Lösung, wird noch 2 h bei 70°C erhitzt. Es folgt mehrstündiges Waschen mit Wasser. Die gewonnene Matrix wird für die Auftrennung von Alkohol Dehydrogenase nach den bekannten Verfahren verwendet.

## Patentansprüche

1. Verfahren zur Herstellung von perl-oder kugelförmigen Partikeln aus PVAL, dadurch gekennzeichnet, daß eine wäßrige PVAL-Lösung, in der ein magnetisches Kolloid dispergiert ist, bei Raumtemperatur in einer mit der Polymerphase nicht mischbaren organischen Phase, die mindestens zwei Emulgatoren enthält, unter Rühren suspendiert wird und während des Suspensionsvorganges durch Zugabe eines wasserlöslichen, mit Hydroxyl-Gruppen reagierenden Agenz vernetzt wird.

2. Verfahren gemäß Anspruch 1, dadurch gekennzeichnet, daß in der organischen Phase 2-6 Vol% Emulgatormischung, bestehend aus mindestens zwei verschiedenen Komponenten, gelöst sind, von denen mindestens eine Komponente partiell wasserlöslich ist.

3. Verfahren gemäß Anspruch 1, dadurch gekennzeichnet, daß in der Polymerphase 0.01 - 2% Gew% eines oder mehrerer Emulgatoren gelöst sind.

4. Verfahren gemäß Anspruch 3, dadurch gekennzeichnet, daß die Emulgatoren Proteine, Cellulose-Derivate, Sulfonsäure-Derivate, Polyvinylpyrrolidon oder Polyäthylenglykole oder Mischungen derselben sind.

5. Verfahren gemäß Anspruch 1, dadurch gekennzeichnet, daß die Polymerlösung 2.5-12.5 Gew% PVAL enthält.

6. Verfahren gemäß Ansprüchen 1-5, dadurch gekennzeichnet, daß der Polymerphase ein magnetisches Kolloid in Form von ferromagnetischen oder superparamagnetischen Substanzen zugemischt wird.

7. Verfahren gemäß Ansprüchen 1-6, dadurch gekennzeichnet, daß 2-7 Vol% Vernetzerlösung, bezogen auf die Polymerphase, zugesetzt werden.

8. Verfahren gemäß Ansprüchen 1-7, dadurch gekennzeichnet, daß der Vernetzer unter Zugabe von wasserlöslichen Diaminen eingesetzt wird.

9. Verfahren gemäß Ansprüchen 1-7, dadurch gekennzeichnet, daß die Vernetzung mit Dialdehyden unter Zusatz von 20-50 Vol % Säure, bezogen auf die Polymerphase, durchgeführt wird.

10. Perl-oder kugelförmige magnetische PVAL Partikel mit Partikelgrößen von 1 - 10 µm, erhältlich nach dem Verfahren gemäß Ansprüchen 1-9.

11. Perl-oder kugelförmige Polymerpartikel nach Anspruch 10, deren Oberfläche chemisch gebundene Polymerketten aufweisen, dadurch gekennzeichnet, daß die die Polymerketten konstituierenden Vinylmonomere Carboxyl-, Hydroxyl-, Amino-, Aldehyd-, oder Oxiran-Gruppen enthalten.

12. Magnetische Partikel gemäß Ansprüchen 10 und 11 , dadurch gekennzeichnet, daß die Oberfläche der Polymerpartikel mit Biomolekülen koppelnde, reaktive Gruppen aufweisen.

13. Perlförmige PVAL Partikel gemäß Anspruch 12, dadurch gekennzeichnet, daß die koppelnden Gruppen mit Antikörpern, Peptiden, Proteinen, Enzymen, Streptavidin, Avidin, Oligonukleotiden oder DNA-Fragmenten umgesetzt sind.

14. Verwendung der PVAL Partikel gemäß Ansprüchen 10-13 zur Fraktionierung von Zellen, Nukleinsäuren, Proteinen, Viren oder Bakterien.

15. Verwendung der PVAL Partikel gemäß Ansprüchen 10-13 für den Immunoassay, für die DNA-Sequenzierung oder DNA-Synthese.

## Claims

1. Process for production of bead-like or spherical particles of PVAL, characterised in that an aqueous PVAL solution in which a magnetic colloid is dispersed is suspended at room temperature by means of stirring in an organic phase not mixable with the polymer phase and containing at least two emulsifying agents, and becomes cross-linked during the suspending process by addition of a water-soluble agent reacting with hydroxyl groups.

2. Process according to claim 1, characterised in that 2-6 vol-% of the emulsifying agent mixture consisting of at least two different components, at least one of which is partially water soluble, is dissolved in the organic phase.

3. Process according to claim 1, characterised in that 0.01 - 2 wt-% of one or more emulsifying agents are dissolved in the polymer phase.

4. Process according to claim 3, characterised in that the emulsifying agents are proteins, derivatives of cellulose, derivatives of sulfonic acid, polyvinyl pyrrolidone or polyethylene glycols of mixtures thereof.

5. Process according to claim 1, characterised in that the polymer solution contains 2.5 - 12.5 wt-% PVAL.

6. Process according to claims 1-5, characterised in that a magnetic colloid, in the form of ferromagnetic or superparamagnetic substances, is admixed to the polymer phase.

7. Process according to claims 1-6, characterised in that 2-7 vol-% cross-linking solution, in relation to the polymer phase, is added.

8. Process according to claims 1-7, characterised in that the cross-linker is employed with addition of water soluble diamines.

9. Process according to claims 1-7, characterised in that the cross-link is carried out with dialdehydes with addition of 20-50 vol-% acid in relation to the polymer phase.

10. Bead-like or spherical magnetic particles of PVAL with particle sizes of 1 - 10 µm obtainable by means of the process according to claims 1-9.

11. Bead-like or spherical polymer particles according to claim 10 whose surfaces have chemically bound polymer chains, characterised in that the vinyl monomers constituting the polymer chains contain carboxyl, hydroxyl, amino, aldehyde or oxirane groups.

12. Magnetic particles according to claims 10 and 11, characterised in that the surface of the polymer particles have reactive groups coupling with biomolecules.

13. Bead-like PVAL particles according to claim 12, characterised in that the coupling groups react with antibodies, peptides, proteins, enzymes, streptavidine, avidine, oligonucleotides or DNA fragments.

14. Use of PVAL particles according to claims 10-13 for fractionating cells, nucleic acids, proteins, viruses or bacteria.

15. Use of PVAL particles according to claims 10-13 for an immunoassay, for DNA sequencing or DNA synthesis.

## Revendications

1. Procédé pour la préparation de particules sous la forme de perles ou sous la forme de billes, constituées de PVAL, caractérisées en ce qu'on met en suspension tout en agitant, à la température ambiante, une solution aqueuse de PVAL dans laquelle un colloïde magnétique a été mis en dispersion, dans une phase organique non miscible avec la phase polymère et qui contient au moins deux émulsifiants, et on procède à une réticulation, au cours du processus de mise en suspension, par addition d'un agent soluble dans l'eau réagissant avec des groupes hydroxyle.

2. Procédé selon la revendication 1, caractérisé en ce que, dans la phase organique, est dissous un mélange d'émulsifiants à raison de 2 à 6 % en volume, constitué par au moins deux composants différents, au moins un de ces composants étant en partie soluble dans l'eau.

3. Procédé selon la revendication 1, caractérisé en ce que, dans la phase polymère, sont présents un ou plusieurs émulsifiants à raison de 0,01 à 2 % en poids.

4. Procédé selon la revendication 3, caractérisé en ce que les émulsifiants sont des protéines, des dérivés de la cellulose, des dérivés d'acide sulfonique, de la polyvinylpyrrolidone ou des polyéthylèneglycols ou encore leurs mélanges.

5. Procédé selon la revendication 1, caractérisé en ce que la solution polymère contient du PVAL à raison de 2,5 à 12,5 % en poids.

6. Procédé selon l'une quelconque des revendications 1 à 5, caractérisé en ce qu'un colloïde magnétique est ajouté par mélange à la phase polymère sous la forme de substances ferromagnétiques ou superparamagnétiques.

7. Procédé selon l'une quelconque des revendications 1 à 6, caractérisé en ce qu'on ajoute une solution d'agent de réticulation à raison de 2 à 7 % en volume rapportés à la phase polymère.

8. Procédé selon l'une quelconque des revendications 1 à 7, caractérisé en ce qu'on met en oeuvre l'agent de réticulation en ajoutant des diamines hydrosolubles.

9. Procédé selon l'une quelconque des revendications 1 à 7, caractérisé en ce qu'on effectue la réticulation avec des dialdéhydes en ajoutant un acide à raison de 20 à 50 % en volume rapportés à la phase polymère.

10. Particules magnétiques de PVAL sous la forme de perles ou de billes possédant des granulométries de 1 à 10 µm, que l'on obtient conformément au procédé selon les revendications 1 à 9.

11. Particules polymères sous la forme de perles ou de billes selon la revendication 10, dont la surface présente des chaînes polymères liées par voie chimique caractérisées en ce que les monomères vinyliques constituant les chaînes polymères contiennent des groupes carboxyle, des groupes hydroxyle, des groupes amino, des groupes aldéhyde ou des groupes oxiranne.

12. Particules magnétiques selon les revendications 10 et 11, caractérisées en ce que la surface des particules polymères présente des groupes aptes à réagir par couplage avec des biomolécules.

13. Particules de PVAL sous la forme de perles selon la revendication 12, caractérisées en ce que les groupes de couplage sont mis à réagir avec des anticorps, avec des peptides, avec des protéines, avec des enzymes, avec de la streptavidine, avec de l'avidine, avec des oligonucléotides ou avec des fragments d'ADN.

14. Utilisation des particules de PVAL selon les revendications 10 à 13 pour le fractionnement de cellules, d'acides nucléiques, de protéines, de virus ou de bactéries.

15. Utilisation des particules de PVAL selon les revendications 10 à 13 pour l'immunodosage, pour le séquençage d'ADN ou pour la synthèse d'ADN.
